(19) Europäisches Patentamt
European Patent Office
Office européen des brevets

(11) Veröffentlichungsnummer: **0 125 576**
**B1**

(12) # EUROPÄISCHE PATENTSCHRIFT

(45) Veröffentlichungstag der Patentschrift: **19.04.89**

(21) Anmeldenummer: **84105024.8**

(22) Anmeldetag: **04.05.84**

(51) Int. Cl.[4]: **C 07 D 501/46**

(54) **Verfahren zur Herstellung von Cephemverbindungen.**

(30) Priorität: **07.05.83 DE 3316797**

(43) Veröffentlichungstag der Anmeldung: **21.11.84 Patentblatt 84/47**

(45) Bekanntmachung des Hinweises auf die Patenterteilung: **19.04.89 Patentblatt 89/16**

(84) Benannte Vertragsstaaten: **AT BE CH DE FR GB IT LI LU NL SE**

(56) Entgegenhaltungen:
**EP-A-0 060 144**
**EP-A-0 070 706**

**Die Akte enthält technische Angaben, die nach dem Eingang der Anmeldung eingereicht wurden und die nicht in dieser Patentschrift enthalten sind.**

(73) Patentinhaber: **HOECHST AKTIENGESELLSCHAFT, Postfach 80 03 20, D-6230 Frankfurt am Main 80 (DE)**

(72) Erfinder: **Kirrstetter, Reiner, Dr., Am Flachsland 56, D-6233 Kelkheim (Taunus) (DE)**
Erfinder: **Dürckheimer, Walter, Dr., Im Lerchenfeld 45, D-6234 Hattersheim am Main (DE)**
Erfinder: **Lattrell, Rudolf, Dr., Heuhohlweg 6H, D-6240 Königstein/Taunus (DE)**
Erfinder: **Schwab, Wilfried, Dr., Am Flachsland 18, D-6233 Kelkheim (Taunus) (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1989

**Beschreibung**

Gegenstand der Erfindung ist ein Verfahren zur Herstellung von Cephemverbindungen der allgemeinen Formel I,

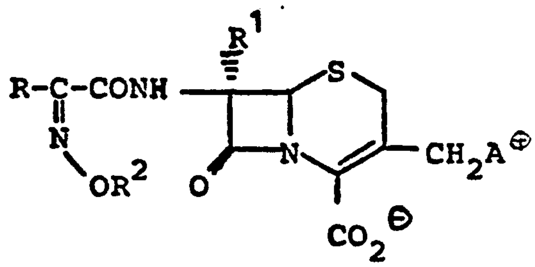

(I)

worin

R einen Thiazolylrest

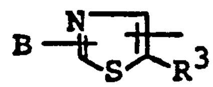

oder einen 1,2,4-Thiadiazolylrest

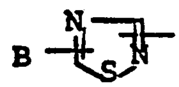

bedeutet, in denen

$R^3$ für Wasserstoff oder Halogen und

B für eine gegebenenfalls substituierte Aminogruppe steht,

und worin

$R^1$ Wasserstoff oder Methoxy,

$R^2$ Wasserstoff, gegebenenfalls substituiertes $C_1$-$C_6$-Alkyl, gegebenenfalls substituiertes $C_2$-$C_6$-Alkenyl, $C_2$-$C_6$-Alkinyl, $C_3$-$C_7$-Cycloalkyl, $C_3$-$C_7$-Cycloalkyl-$C_1$-$C_6$-alkyl, $C_4$-$C_7$-Cycloalkenyl, die Gruppe

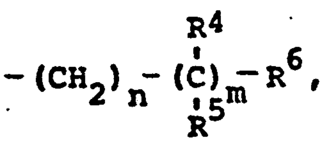, worin

m und n jeweils für 0 oder 1 steht,

$R^4$ und $R^5$ gleich oder verschieden sein können und Wasserstoff, Aryl oder eine $C_1$-$C_4$-Alkylgruppe bedeuten, oder zusammen mit dem Kohlenstoffatom, an das sie gebunden sind, eine Methylen- oder eine $C_3$-$C_7$-Cycloalkylidengruppe bilden, wobei die $C_1$-$C_4$-Alkyl- und die $C_3$-$C_7$-Cycloalkylidengruppe noch weiter ein- oder mehrfach substituiert sein können,

$R^6$ eine COOH-, CN- oder $CONH_2$-Gruppe, wobei letztere am Stickstoff ein oder zweimal substituiert sein kann, einen Chinolinium-

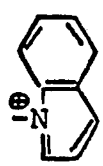

oder einen Isochinoliniumrest

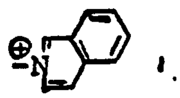

die jeweils auch ein- oder mehrfach, gleich oder verschieden substituiert sein können durch gegebenenfalls substituiertes $C_1$-$C_6$-Alkyl, $C_1$-$C_6$-Alkoxy, Halogen, Trifluormethyl oder Hydroxy; oder einen Phenanthridiniumrest, oder einen Pyridiniumrest

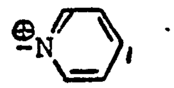

der ein- oder mehrfach, gleich oder verschieden substituiert sein kann durch gegebenenfalls substituiertes $C_1$-$C_6$-Alkyl, wobei 2 orthoständige Alkylgruppen auch zu einem gegebenenfalls substituierten Di- bis Decamethylenring geschlossen sein können, in dem ein Ring-C-Atom durch ein Heteroatom ersetzt sein kann und weiterhin auch noch eine oder zwei Doppelbindungen enthalten sein können;

durch gegebenenfalls substituiertes $C_2$-$C_6$-Alkenyl,
durch $C_2$-$C_6$-Alkinyl,
durch $C_3$-$C_7$-Cycloalkyl oder $C_3$-$C_7$-Cycloalkylmethyl, wobei in beiden Substituenten der Ring auch substituiert sein kann;
durch $C_4$-$C_7$-Cycloalkenyl,
durch gegebenenfalls substituiertes $C_1$-$C_6$-Alkoxy,
durch $C_2$-$C_6$-Alkenyloxy oder $C_2$-$C_6$-Alkinyloxy,
durch Halogen, Trifluormethyl oder Hydroxy,
durch gegebenenfalls substituiertes Phenyl, Benzyl oder Heteroaryl,
durch Formyl oder ketalisiertes Formyl,
durch gegebenenfalls substituiertes $C_1$-$C_6$-Alkylcarbonyl, das auch in ketalisierter Form vorliegen kann,
durch Arylcarbonyl,
durch Carbamoyl,
bedeutet und in der die $R^2O$-Gruppe in syn-Position steht.

Die Erfindung ist insbesondere auf die Herstellung von Verbindungen gerichtet, in denen
R und $R^1$ die vorstehenden Bedeutungen besitzen und
B eine Aminogruppe, die substituiert sein kann durch Aminoschutzgruppen,
$R^2$ Wasserstoff, $C_1$-$C_6$-Alkyl, das ein- oder mehrfach substituiert sein kann durch Halogen, $C_1$-$C_6$-Alkylthio, $C_1$-$C_6$-Alkyloxy, Aryl oder Heteroaryl, $C_2$-$C_6$-Alkenyl, das ein- oder mehrfach substituiert sein kann durch Halogen; $C_2$-$C_3$-Alkinyl, $C_3$-$C_6$-Cycloalkyl, $C_3$-$C_6$-Cycloalkyl-$C_1$-$C_6$-alkyl, $C_4$-$C_7$-Cycloalkenyl und in der die Gruppe

$$\text{Gruppe}\ -(CH_2)_n-\underset{R^5}{\overset{R^4}{(C)}}{}_m-R^6$$

die vorstehende Bedeutung hat,
A einen Chinolinium- oder einen Isochinoliniumrest, die jeweils auch ein- oder mehrfach, gleich oder verschieden substituiert sein können durch $C_1$-$C_6$-Alkyl, das substituiert sein kann
durch Hydroxy,
durch $C_1$-$C_6$-Alkoxy,
durch Halogen,
durch Trifluormethyl,
durch Hydroxy,
oder einen Pyridiniumrest

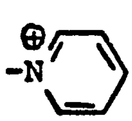

bedeuten, der ein- oder mehrfach, gleich oder verschieden substituiert sein kann durch $C_1$-$C_6$-Alkyl, das ein- oder mehrfach substituiert sein kann durch Hydroxy; Formyl oder $C_1$-$C_6$-Alkylcarbonyl, deren Carbonylgruppen auch in ketalisierter Form vorliegen können; Sulfo, Carbamoyl, $C_1$-$C_6$-Alkyloxy, Hydroxy-$C_1$-$C_6$-alkyloxy und wobei 2 Alkylgruppen auch zu einem gegebenenfalls substituierten Di- bis Decamethylen-Ring geschlossen

sein können, in dem ein Ring-C-Atom durch ein Heteroatom ersetzt sein kann und weiterhin auch noch eine oder zwei Doppelbindungen enthalten sein können,

durch $C_2$-$C_6$-Alkenyl, das durch Hydroxy substituiert sein kann,
durch $C_2$-$C_6$-Alkinyl,
durch $C_3$-$C_7$-Cycloalkyl oder $C_3$-$C_7$-Cycloalkyl-methyl,
wobei in beiden Substituenten der Ring auch substituiert sein kann durch Hydroxy oder Halogen,
durch $C_4$-$C_7$-Cycloalkenyl,
durch $C_1$-$C_6$-Alkoxy, das durch Hydroxy substituiert sein kann,
durch $C_2$-$C_6$-Alkenyloxy oder $C_2$-$C_6$-Alkinyloxy,
durch Halogen, Trifluormethyl oder Hydroxy,
durch Phenyl, Benzyl oder Heteroaryl, die auch durch Halogen substituiert sein können,
durch Formyl oder ketalisiertes Formyl,
durch $C_1$-$C_6$-Alkylcarbonyl, das auch durch Hydroxy substituiert sein und auch in ketalisierter Form vorliegen kann,
durch Arylcarbonyl,
durch Carbamoyl,

und wobei auch bei diesen bevorzugten, unter die allgemeine Formel I fallenden Verbindungen die $R^2O$-Gruppe in syn-Position steht.

Als gegebenenfalls mögliche Substituenten des unter A erwähnten Di- bis Decamethylen-Rings, in dem ein Ring-C-Atom durch ein Heteroatom ersetzt sein kann und weiterhin auch noch eine oder zwei Doppelbindungen enthalten sein können, kommen insbesondere die folgenden Substituenten in Betracht, die ein- oder mehrfach, vorzugsweise jedoch einfach, auftreten können:

$C_1$-$C_6$-Alkyl, $C_1$-$C_4$-Alkoxy, Hydroxymethyl, Halogen, Hydroxy, Oxo, Exomethylen.

Diese Substituenten können an den genannten, an den Pyridiniumrest ankondensierten Ringen auftreten, unabhängig davon, ob der jeweilige Ring gesättigt, ungesättigt oder auch noch durch ein Heteroatom unterbrochen ist.

Der an den Pyridiniumrest ankondensierte Ring kann 2 bis 10 Ringglieder (Di- bis Decamethylen), vorzugsweise jedoch 3 bis 5 Ringglieder enthalten und somit beispielsweise einen Cyclopenteno-, Cyclohexeno- oder Cyclohepteno-Ring darstellen. Enthält ein solcher ankondensierter Ring eine Doppelbindung, so seien als Beispiele ein Dehydrocyclopentadieno-, Dehydrocyclohexadieno- oder Dehydrocycloheptadieno-Ring genannt. Ist in derartigen Ringen ein C-Atom durch ein Heteroatom ersetzt, so kommen als Heteroatome insbesondere Sauerstoff oder Schwefel in Betracht. Als ein Sauerstoffatom enthaltende, ankondensierte Ringe, die zwei oder eine Doppelbindung enthalten, seien beispielsweise erwähnt Furo, Pyrano, Dihydrofuro und Dihydropyrano; als ankondensierte Ringe mit einem Schwefelatom, die zwei oder eine Doppelbindung enthalten, Thieno, Thiopyrano, Dihydrothieno und Dihydrothiopyrano. Von den ein Heteroatom enthaltenden, ankondensierten Ringen kommen für eine Substitution, insbesondere durch die vorstehend angegebenen Substituenten, insbesondere diejenigen Ringe in Betracht, die nur eine Doppelbindung enthalten.

Als besonders bevorzugt kommen beispielsweise die folgenden Substituenten in Betracht:

B: $NH_2$, HCONH, $CF_3CONH$, $CCl_3CONH$, $C_6H_5CH_2CONH$, $(C_6H_5)_3CNH$, $HSO_3NH$, $(CH_3)_2\,CH{=}N$,

R:

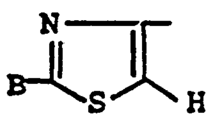 , 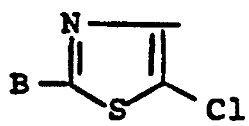 , 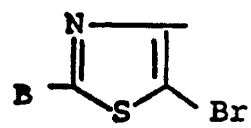 ,

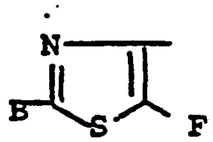 , 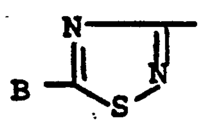

$R^1$: Wasserstoff, $OCH_3$

$R^2$: Wasserstoff, $C_1$-$C_6$-Alkyl, wie z. B. Methyl, Äthyl, Propyl, Isopropyl, Butyl, vorzugsweise Methyl, Äthyl; $C_1$-$C_2$-Halogenalkyl, z. B. Chlor, Brom, Jod oder Fluor-substituiertes Alkyl, vorzugsweise Trifluorethyl, Difluormethyl, 2,2,3,3-Tetrafluorpropyl, durch Aryl, wie z. B. Phenyl, Tolyl, Chlorphenyl substituiertes Alkyl, insbesondere Benzyl,

durch Heteroaryl substituiertes Alkyl, wie z. B. 1,3-Thiazol-4-yl-substituiertes Alkyl, insbesondere 1,3-Thiazol-4-yl-methyl,

$C_2$-$C_6$-Alkenyl, wie z. B. Vinyl, Allyl, Isopropenyl, Methallyl, insbesondere Allyl, Methallyl,

durch Halogen, wie z. B. Chlor oder Brom-substituiertes $C_2$-$C_6$-Alkenyl, insbesondere 3-Chlor-propen-2-yl, 2-Brom-propen-2-yl, 2-Chlorpropen-2-yl;

$C_2$-$C_3$-Alkinyl, wie insbesondere Propargyl,

$C_3$-$C_7$-Cycloalkyl, wie insbesondere Cyclopropyl, Cyclobutyl, Cyclopentyl,

$C_3$-$C_7$-Cycloalkyl-$C_1$-$C_6$-alkyl wie insbesondere Cyclopropylmethyl, Cyclobutylmethyl, $C_4$-$C_7$-Cycloalkenyl wie insbesondere Cyclopenten-1-yl, die Gruppe

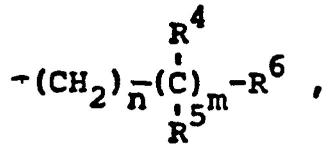

wobei $R^4$ und $R^5$ gleich oder verschieden sein können und Wasserstoff, Aryl, vorzugsweise Phenyl, $C_1$-$C_4$-Alkyl, wie z. B. Methyl, Ethyl, Propyl, Isopropyl, Butyl, sec. Butyl, vorzugsweise Methyl, Ethyl, insbesondere Methyl, bedeuten könneh, oder

wobei $R^4$ und $R^5$ zusammen mit dem Kohlenstoffatom, an das sie gebunden sind, eine Methylengruppe oder eine $C_3$-$C_7$-Cycloalkylidengruppe bilden können, wie z. B. Cyclopropyl, Cyclobutyl, Cyclopentyl, Cyclohexyl, Cycloheptyl, vorzugsweise Cyclopropyl, Cyclobutyl, Cyclopentyl oder Cyclohexyl, und wobei die Cycloalkylidengruppe substituiert sein kann, z. B. durch $C_1$-$C_4$-Alkyl, vorzugsweise Methyl, durch Halogen, vorzugsweise Fluor und Chlor, oder auch substituiert sein kann durch Alkylen mit 3 - 6 C-Atomen,

m = 0 oder 1

n = 0 oder 1, wobei die Summe von m uhd n 1 oder 2 darstellt.

Bevorzugte Beispiele für die Gruppe

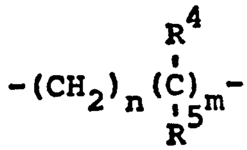

sind die folgenden:

Für den Fall, daß n = 0 und m = 1 ist:

$-CH(CH_3)$, $-C(CH_3)_2$, $-CH(C_6H_5)$,

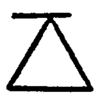 , 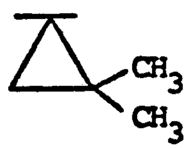 , 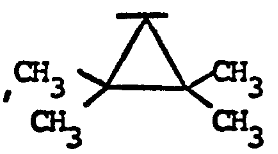 , 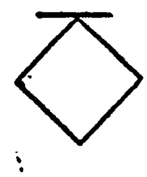

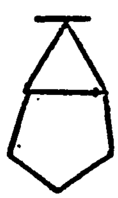 , 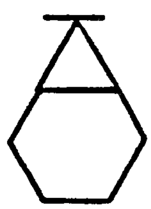 , 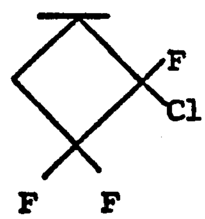 , 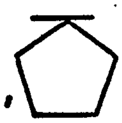 , 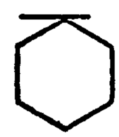

für den Fall, daß m = 0 und n = 1 ist: $-CH_2-$ und falls n und m = 1 sind: $-CH_2-C(=CH_2)-$.

$R^6$ die Gruppe COOH, CN, $CONH_2$, durch $C_1$-$C_6$-Alkyl, vorzugsweise Methyl oder Ethyl, substituiertes Carbamoyl.

A ein Chinolinium- oder ein Isochinoliniumrest, die jeweils auch ein- oder mehrfach, gleich oder verschieden substituiert sein können durch $C_1$-$C_6$-Alkyl, wie z. B. Methyl, Ethyl, Propyl, Isopropyl, vorzugsweise Methyl, durch Methoxy, durch Hydroxy, durch Halogen oder Trifluormethyl, ein Pyridiniumrest, der ein- oder mehrfach, vorzugsweise 1- bis 3-fach, insbesondere 1- bis 2-fach substituiert sein kann, beispielsweise durch $C_1$-$C_4$-Alkyl, wie insbesondere Methyl, Ethyl, Propyl, Isopropyl, n-Butyl, sec.-Butyl, tert.-Butyl, Dimethyl, Trimethyl, Methyl und Ethyl, Methyl und Propyl, Methyl und Isopropyl, Ethyl und Ethyl; Hydroxy-$C_1$-$C_4$-alkyl, wie insbesondere Hydroxymethyl, Hydroxyethyl, Hydroxypropyl, Hydroxyisopropyl, Hydroxybutyl, Hydroxy-sec.-butyl oder Hydroxy-tert.-butyl, und wobei z. B. auch zwei oder drei Hydroxygruppen am Alkylrest stehen können; Formyl-$C_1$-$C_4$-alkyl, wie insbesondere Formylmethyl, $C_1$-$C_4$-Alkyl-carbonyl-$C_1$-$C_4$-alkyl, wie insbesondere Methylcarbonylmethyl, Ethylcarbonylmethyl, Methylcarbonylethyl und Ethylcarbonylethyl; $C_3$-$C_4$-Alkenyl, wie insbesondere Allyl, 2-Methylallyl und Buten-3-yl, die auch noch durch Hydroxy substituiert sein können, wie insbesondere Hydroxyallyl und Hydroxybutenyl; $C_3$-Alkinyl, wie insbesondere Propargyl; $C_3$-$C_6$-Cycloalkyl und

$C_3$-$C_6$-Cycloalkyl-methyl, wobei sich die Kohlenstoffzahl auf den Cycloalkylteil bezieht, wie insbesondere Cyclopropyl, Cyclobutyl, Cyclopentyl, Cyclohexyl und Cyclopentylmethyl, wobei die Ringe auch substituiert sein können, z. B. durch Hydroxy, wie insbesondere 1-Hydroxy-1-cyclopentyl und 1-Hydroxy-1-cyclohexyl, oder durch Halogen, vorzugsweise Chlor;

$C_5$-$C_6$-Cycloalkenyl, wie insbesondere Cyclopenten-1-yl und Cyclohexen-1-yl;

$C_1$-$C_6$-Alkoxy, wie insbesondere Methyl- und Ethyloxy, Halogen, wie insbesondere 3-Fluor, 3-Chlor, 3-Brom, 3-Jod; Hydroxy; insbesondere 3-Hydroxy; Trifluormethyl, insbesondere 3-Trifluormethyl; Phenyl und Benzyl, die auch substituiert sein können, beispielsweise durch Halogen, insbesondere Chlor, wie z. B. 4-Chlorbenzyl; 2'-Thienyl und 3'-Thienyl;

$C_1$-$C_4$-Alkylcarbonyl, insbesondere Acetyl und Propionyl, vorzugsweise Acetyl; Formyl, Benzoyl, Carbamoyl.

Stellt A einen Pyridiniumrest dar, der durch zwei zu einem Di- bis Decamethylen-Ring geschlossenen Alkylgruppen substituiert ist, der wiederum ein- oder mehrfach, vorzugsweise einfach substituiert sein und eine oder zwei Doppelbindungen enthalten kann, so kommen hierfür ganz besonders die folgenden ankondensierten Ringsysteme in Betracht:

Cyclobuteno, Cyclopenteno, Hydroxycyclopenteno, Oxocyclopenteno, Hydroxymethylcyclopenteno, Exomethylencyclopenteno, Carboxycyclopenteno und Carbamoylcyclopenteno, Cyclohexeno, Hydroxycyclohexeno, Oxocyclohexeno, Hydroxymethylcyclohexeno, Exomethylencyclohexeno, Carboxycyclohexeno und Carbamoylcyclohexeno, Cyclohepteno, Hydroxy-, Oxo-, Hydroxymethyl, Exomethylen-, Carboxycyclohepteno und Carbamoylcyclohepteno, Dehydrocyclopenteno, Dehydrocyclohexeno und Dehydrocyclohepteno.

Ist in den vorstehend genannten ankondensierten Ringsystemen ein Ring-C-Atom durch ein Heteroatom, insbesondere Sauerstoff oder Schwefel ersetzt, so kommen insbesondere die folgenden Ringsysteme in Betracht:

Furo[2,3-b]pyridin, Furo[3,2-b]pyridin, Furo[2,3-c]pyridin, Furo[3,2-c]pyridin, Thieno[2,3-b]pyridin, Thieno[3,2-b]pyridin, Thieno[2,3-c]pyridin, Thieno[3,2-c]pyridin, Thieno[3,4-b]pyridin, Thieno[3,4-c]pyridin.

Das erfindungsgemäße Verfahren zur Herstellung von Verbindungen der allgemeinen Formel I ist dadurch gekennzeichnet, daß man in einem Eintopf-Verfahren ohne Bildung des aus der EP-A-0 060 144 bekannten 3-Jodmethylderivats eine Verbindung der allgemeinen Formel II

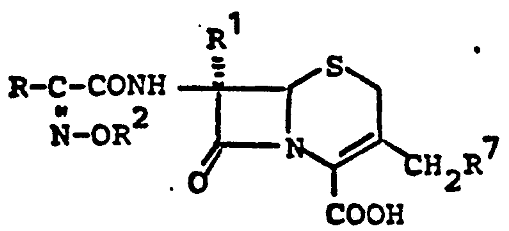

(II)

oder deren Salze, worin

R, $R^1$ und $R^2$ die in der allgemeinen Formel I genannte Bedeutung haben, und

$R^7$ eine Acetoxy-, Chloracetoxy-, Acetylacetoxy- oder Carbamoyloxygruppe bedeutet, mit einer Base, die den Resten A der allgemeinen Formel I entspricht und in Gegenwart von Tri-$C_1$-$C_4$-alkyljodsilan, vorzugsweise Trimethyl- oder Triethyljodsilan, umsetzt und

a) eine gegebenenfalls vorhandene Schutzgruppe abspaltet und

b) falls erforderlich, das so erhaltene Produkt in ein physiologisch verträgliches Säureadditionssalz überführt.

Besonders bevorzugt ist die Verwendung von Trimethyljodsilan.

Die Ausgangsverbindungen sind literaturbekannt oder können nach literaturbekannten Verfahren hergestellt werden (vgl. z. B. DE-OS-2 716 707, DE-OS-3 118 732, deutsche Patentanmeldungen P 3 207 840, P 3 247 613, P 3 247 614).

Es ist aus EP-A-0 064 740 sowie P 3 207 840 bekannt, daß Verbindungen der allgemeinen Formel I, in der R einen 2-Aminothiazol-4-ylrest bedeutet, und ihre physiologisch verträglichen Salze ausgezeichnete antibakterielle Wirksamkeiten sowohl gegen grampositive als auch gramnegative bakterielle Keime besitzen. Diese Verbindungen lassen sich beispielsweise aus Verbindungen der allgemeinen Formel II durch direkte Umsetzung mit den entsprechenden Basen, vorzugsweise in Wasser oder wäßrigen Gemischen als Lösungsmittel, herstellen. Weiterhin ist in der Literatur (EP-A-0 060 144) beschrieben, daß 3-Jodmethylcephalosporinderivate, z. B. Verbindungen, die denen der allgemeinen Formel I mit A = J, entsprechen, mit Pyridinbasen zu den entsprechenden Pyridiniumverbindungen reagieren. Derartige Jodalkylverbindungen lassen sich allgemein aus Estern, z. B. Acetaten, mit Trimethyljodsilan herstellen [J. Amer. Chem. Soc. 99, 968, (1977); Angew. Chemie 91, 648, (1979)], eine Reaktion, die in der Folgezeit auf Cephalosporine übertragen worden ist [vgl. EP-A-0 034 924, US-PS-4 266 049, Tetrahedron Letters 1981, 3915, EP-A-0 070 706 (Beispiel 5)].

Nach dem in der EP-A-0 060 144 beschriebenen Zweistufenverfahren werden z. B. die den der allgemeinen Formel II entsprechenden Acetate ($R^7$ = $OCOCH_3$) zuerst in die 3-Jodmethylverbindungen übergeführt, diese isoliert und dann mit den gewünschten Pyridinbasen zur Reaktion gebracht. Zur Isolierung der Endprodukte ist eine chromatographische Reinigung nötig. Die maximale Ausbeute an reinem Endprodukt liegt unter 10 % d.

Th.

Überraschenderweise wurde nun gefunden, daß nach dem erfindungsgemäßen Verfahren die Ausbeuten an den Endprodukten der allgemeinen Formel I entscheidend bis zum mehr als zehnfachen erhöht werden, wenn die nucleophile Austauschreaktion von Beginn an in Gegenwart eines Überschusses der entsprechenden, dem Rest A in der allgemeinen Formel I zugrundeliegenden Basen ausgeführt wird, d.h. ein Tri-$C_1$-$C_4$-alkyljodsilan, vorzugsweise Trimethyljodsilan, nach Zugabe der Base zur Reaktionsmischung gegeben wird.

Das Verfahren wird so durchgeführt, daß zu einer Lösung oder Suspension der Verbindung II in einem geeigneten Lösungsmittel die dem Rest A entsprechende Base zugegeben wird, gefolgt von Trimethyljodsilan. Statt Trimethyljodsilan kann beispielsweise auch eine Reaktionsmischung aus Jod und Hexamethyldisilan, die vorher bei Temperaturen zwischen etwa 60 und 120°C in literaturbekannter Weise zur Reaktion gebracht wurde, wobei Trimethyljodsilan entsteht, verwendet werden. Statt Trimethyljodsilan kann mit demselben guten Ergebnis auch Triethyljodsilan, das in literaturbekannter Weise hergestellt wird, verwendet werden.

Die Reaktion wird ausgeführt bei Temperaturen zwischen etwa -5 und +100°C, vorzugsweise zwischen +10 und +80°C.

Geeignete inerte aprotonische Lösungsmittel sind z. B. chlorierte Kohlenwasserstoffe, wie Methylenchlorid, Chloroform, Dichlorethan, Trichlorethan, Tetrachlorkohlenstoff, oder Niederalkylnitrile, wie Acetonitril oder Propionitril, oder Frigene, oder Toluol; insbesondere ist Methylenchlorid ein hervorragendes Lösungsmittel.

Die dem Rest A entsprechende Base wird in mindestens stöchiometrischer Menge bis zu einem zwanzigfachen Überschuß zugegeben, vorzugsweise wird mit solchen Mengen gearbeitet, daß die freiwerdende Jodwasserstoffmenge gebunden wird und noch mindestens 1 Mol, vorzugsweise 2 - 5 Mol der Base für die Substitution zur Verfügung stehen.

Da neben der auszutauschenden Gruppe $R^7$ in der Ausgangsverbindung II auch andere vorliegende funktionelle Gruppen, wie Amino-, Carboxy- oder Amidgruppen, mit Trimethyljodsilan reagieren, wird letzteres in mindestens vierfachem bis zu etwa zwanzigfachem, vorzugsweise in fünf- bis zehnfachem Überschuß zugegeben.

Das Verfahren zur Bildung der Cephalosporine der allgemeinen Formel I läßt sich auch so durchführen, daß ein Gemisch aus dem Rest A entsprechender Base und Trimethyljodsilan zu einer Suspension der Verbindung II in einem geeigneten Lösungsmittel, wie z. B. Methylenchlorid, gegeben wird oder auch umgekehrt, und dann die Reaktion erfolgt. Eine weitere Variante besteht darin, daß die Reaktion nach der Zusammengabe der Reaktionskomponenten in den oberen beschriebenen Weisen in einem abgeschlossenen System, wie z. B. einem Autoklaven, bei Temperaturen zwischen 10 und 100°C durchgeführt werden kann. Nach Abkühlen auf 0 - 20°C und Entlüften des abgeschlossenen Systems wird in der üblichen Weise aufgearbeitet.

Carboxyl- und N-Aminofunktionen in der Verbindung II können auch durch Zugabe eines Silylierungsmittels, wie z. B. Bistrimethylsilylacetamid, Bistrimethylsilyltrifluoroacetamid, Trimethylchlorsilan, Hexamethyldisilazan, Bistrimethylsilylharnstoff, vorsilyliert werden, entweder ohne oder in Gegenwart einer Base, vorzugsweise der gewünschten, der Gruppe A zugrundeliegenden Base in den vorstehend beschriebenen Mengen. Anschließend wird Trimethyljodsilan in mindestens stöchiometrischer Menge oder auch im Überschuß, vorzugsweise in einem doppelten bis zu einem zehnfachen Überschuß zugegeben.

Wenn in der dem Rest A in der allgemeinen Formel I zugrundeliegenden Base funktionelle Gruppen, wie z. B. Hydroxygruppen und dergleichen, vorliegen, so werden diese vorzugsweise mit einem der vorstehend genannten Silylierungsmittel vorsilyliert und sodann in der Reaktion eingesetzt.

Die Reaktionsprodukte der allgemeinen Formel I können beispielsweise durch Zugabe von Wasser oder wäßrigen Mineralsäuren, z. B. verdünnte HCl, HBr, HJ oder $H_2SO_4$, aus der wäßrigen Phase in üblicher Weise, z. B. durch Gefriertrocknen der Wasserphase, Chromatographie und dergleichen isoliert werden. Vorzugsweise werden die polaren Reaktionsprodukte aus der Reaktionslösung durch Zugabe von wäßrigen Mineralsäuren, wie z. B. HCl, HBr, HJ oder $H_2SO_4$, gelöst in Alkoholen, wie z. B. Methanol, Ethanol, Propanol, Isopropanol, Butanol, Isobutanol oder Aceton oder deren Gemische, als schwerlösliche Salze ausgefällt.

Diese werden sodann nach literaturbekannten Verfahren, z. B. nach der EP-A-0 112 550 in physiologisch verträgliche Säureadditionssalze überführt.

Die folgenden Ausführungsbeispiele für die nach dem erfindungsgemäßen Verfahren herstellbaren Verbindungen dienen zur weiteren Erläuterung der Erfindung, schränken sie jedoch nicht darauf ein.

**Beispiel 1:**

<u>1-[2-(2-Aminothiazol-4-yl)-2-syn-methoxyimino-acetamido)-3-[(2,3-cyclopenteno-1-pyridinio)methyl]-ceph-3-em-4-carboxylat-dihydrojodid</u>

<u>Variante a:</u>

Zu einer Mischung aus 45,5 g (0,1 mol). 1-[2-(2-Aminothia-zol-4-yl)-2-syn-methoxyimino-acetamido]-cephalosporansäure und 900 ml Methylendichlorid werden 100 ml (0,85 mol) 2,3-Cyclopentenopyridin und sodann 140 g (100 ml, 0,7 mol) Trimethyljodsilan gegeben. Die rotbraun gefärbte Lösung wird 2 Stunden unter Rückfluß gehalten, dann auf -15°C gekühlt und unter Umschütteln mit einer Lösung von 60 g Kaliumjodid in 250 ml 2n HCl versetzt. Es bildet sich ein Niederschlag, der unter gelegentlichem Umschütteln 2 Stunden im Eisbad und sodann über Nacht im Kühlschrank belassen wird. Es wird abgesaugt, der gelbe Niederschlag dreimal mit

je 100 ml Eiswasser im Becherglas verrührt und jeweils abgesaugt. Das feuchte Produkt wird sodann unter Rühren in 500 ml Aceton eingetragen, abgesaugt und dreimal mit je 100 ml Aceton gewaschen. Nach dem Trocknen i. Vak. über $H_2SO_4$ erhält man 54,5 g (69 % d. Th.) hellgelbgefärbte Kristalle vom Zers.-punkt 179 - 181°C.

$C_{22}H_{22}N_6O_5S_2$ x 2HJ x $H_2O$ (788,43)
Ber.: C 33,51, H 3,32, J 32,19, N 10,66, S 8,13, $H_2O$ 2,3 %
Gef.: C 33,6 H, 3,6, J 31,3, N 10,7, S 7,1, $H_2O$ 2,5 %
IR (KBr): 1785 $cm^{-1}$ (Lactam-CO).
$^1$H-NMR($CF_3CO_2D$): δ = 2,30 - 2,85 (m, 2H, Cyclopenten-H); 3,10 - 4,05 (m, 6H, 4 Cyclopenten-H und $SCH_2$); 4,41 (s, 3H, $OCH_3$); 5,25 - 6,23 (m, 4H, $CH_2$Py und 2 Lactam-H); 8,11 (s, 1H, Thiazol); 7,65 - 8,70 ppm (m, 3H, Py).

Variante b:

58,5 g (82 ml, 0,4 mol) Hexamethyldisilan werden auf 70 - 75°C erhitzt und portionsweise 88,8 g (0,7 mol) Jod innerhalb von 20 Min. zugegeben. Nach der Jodzugabe wird 30 Min. unter Rückfluß gehalten, auf 10°C gekühlt und mit 900 ml Methylendichlorid verdünnt. Sodann werden 100 ml (0,85 mol) 2,3-Cyclopentenopyridin, gefolgt von 45,5 g (0,1 mol) 7-[2-(2-Aminothiazol-4-yl)-2-syn-methoxyimino-acetamido]-cephalosporansäure zugegeben. Die Mischung wird 2 Stunden unter Rückfluß erhitzt und anschließend wie vorstehend mit KJ/2n HCl hydrolysiert. Der gebildete Niederschlag wird wie bei Variante a beschrieben isoliert. Ausbeute 57,5 g (73 % d. Th) hellgelbes Dihydrojodidsalz. Die Verbindung ist in allen Eigenschaften mit der vorstehend beschriebenen identisch.

Variante c:

Zu einer Lösung von 4,6 g (36 mmol) Jod in 35 ml Methylendichlorid werden 3,2 ml (20 mmol) Triäthylsilan gegeben und die Lösung 30 Min. unter Rückfluß erhitzt. Es wird gekühlt, 1,45 ml (12 mmol) 2,3-Cyclopentenopyridin und sodann 0,91 g (2 mmol) 7-[2-(2-Aminothiazol-4-yl)-2-syn-methoxyimino-acetamido]-cephalosporansäure zugegeben. Die rotbraungefärbte Lösung wird 2 Stunden unter Rückfluß erhitzt, auf -20°C gekühlt und durch Zugabe einer Lösung von 2 g Kaliumjodid in 10 ml 2n HCl hydrolysiert. Die Mischung wird 4 Stunden im Eisbad gerührt, über Nacht im Kühlschrank belassen, der Niederschlag abgesaugt und dreimal mit je 5 ml Eiswasser sowie dreimal mit je 10 ml Aceton gewaschen. Nach dem Trocknen erhält män 1,1 g (70 % d. Th) hellgelbe Kristalle.

Die Verbindung ist in allen Eigenschaften mit der unter Variante a beschriebenen identisch.

**Beispiel 2:**

3-[2,3-Cyclopenteno-1-pyridinio)methyl]-7-[2-syn-methoxyimino-2-(2-phenylacetamido-thiazol-4-yl)acetamido]-ceph-3-em-4-carboxylat-hydrojodid.

Aus 0,86 g (1,5 mmol) 7-[2-syn-Methoxyimino-2-(2-phenyl-acetamido-thiazol-4-yl)acetamido]-cephalosporansäure, 1,1 ml (9 mmol) 2,3-Cyclopentenopyridin und 1,1 ml (7,5 mmol) Trimethyljodsilan in 35 ml Methylendichlorid in Analogie zu Beispiel 1a. Nach der Hydrolyse mit 40 ml 2n HCl bei 5°C wird der ausgefallene gelbe Niederschlag abgesaugt, mit wenig Eiswasser gewaschen und über $P_2O_5$ getrocknet.
Ausbeute 1,0 g (88 % d. Th) hellgelber Feststoff.
IR(KBr): 1785 $cm^{-1}$ (Lactam -CO)
$^1$H-NMR ($D_6$-DMSO): δ = 1,8 - 2,4 (m, 2H, Cydopenten-H); 2,8 - 3,5 (m, 6H, 4 Cydopenten-H und $SCH_2$); 3,72 (s, 2H, $CH_2$-CO); 3,83 (s, 3H, $OCH_3$); 5,17 (d, 1H, Lactam-H); 5,48 (breites s, 2H, ($CH_2$Py); 5,85 (q, 1H, Lactam-H); 7,27 (s, 5H, $C_6H_5$); 7,5 - 8,8 (m, 4H, Py und Thiazol); 9,67 (d, 1H, NH); 12,70 ppm (s, 1H,NH).

**Beispiel 3:**

3-[(2,3-Cyclopenteno-1-pyridinio)methyl]-7-[2-syn-methoxy-imino-2-(2-tritylamino-thiazol-4-yl)acetamido]-ceph-3-em-4-carboxylat-hydrojodid.

Aus 1,16 g (1,5 mmol) 7-[2-syn-Methoxyimino-2-(2-tritylamino-thiazol-4-yl)acetamido]-cephalosporansäure, 1,1 ml (9 mmol) 2,3-Cyclopentenopyridin und 1,1 ml (7,5 mmol) Trimethyljodsilan in 35 ml Methylendichlorid in Analogie zu Beispiel 1a. Nach der Hydrolyse mit 40 ml 2n HCl bei 5°C wird am Rotationsvakuumverdampfer das Methylendichlorid entfernt, die Wasserphase von einem öligen Rückstand abdekantiert und der Rückstand 2 Stunden mit 40 ml Wasser bei 0 - 5°C verrührt. Der gebildete Niederschlag wird abgesaugt, mit Eiswasser gewaschen und über $P_2O_5$ getrocknet.
Ausbeute: 1,24 g (93 % d. Th) gelber Feststoff.
IR (KBr): 1785 $cm^{-1}$ (Lactam-CO)
$^1$H-NMR ($D_6$-DMSO): δ = 1,8 - 2,4 (m, 2H, Cyclopenten-H); 2,9 - 3,5 (m, 6H, 4 Cyclopenten-H und $SCH_2$); 3,80 (s, 3H, $OCH_3$); 5,16 (d, 1H, Lactam-H); 5,50 (breites s, 2H, $CH_2$Py); 5,74 (q, 1H, Lactam-H); 6,70 (s, 1H, Thiazol); 7,30 (s, 15H, $C_6H_5$); 7,7 - 8,8 (m, 3H, Py); 9,0 (breites s, 1H, NH); 9,58 ppm (d, 1H, NH).

**Beispiel 4:**

7-[2-(2-Aminothiazol-4-yl)-2-syn-methoxyimino-acetamido]-3-(1-pyridiniomethyl)-ceph-3-em-4-carboxylat-dihydrojodid.

Aus 4,55 g (10 mmol) 7-[2-(2-Aminothiazol-4-yl)-2-syn-methoxyimino-acetamido]-cephalosporansäure, 6,8 ml (8,5 mmol) Pyridin und 10 ml (70 mmol) Trimethyljodsilan in 100 ml Methylendichlorid in Analogie zu Beispiel 1a. Nach dem Erkalten wird die rotbraune Lösung mit einer Lösung von 10 g KJ in 50 ml 2n HCl hydrolysiert, das Gemisch 4 Stunden im Eisbad gerührt und über Nacht im Kühlschrank belassen. Der Niederschlag wird filtriert und viermal mit wenig Eiswasser gewaschen. Nach dem Trocknen über $P_2O_5$ erhält man 4,5 g (61 % d. Th) der Titelverbindung in Form eines gelben Feststoffes.

IR(KBr):1783 $cm^{-1}$ (Lactam-CO)

$^1H$-NMR($CF_3CO_2D$): $\delta$ = 3,53 und 3,95 (AB,J = 19Hz, 2H, $SCH_2$); 4,40 (s, 3H, $OCH_3$). 5,2 - 6,4 (m, 4H, $CH_2Py$ und 2 Lactam-H); 7,9 - 9,3 ppm (m, 6H, Py und Thiazol).

**Beispiel 5:**

7-[2-(2-Aminothiazol-4-yl)-2-syn-methoxyimino-acetamido]-3-(5-phenanthidiniomethyl)-ceph-3-em-4-carboxylat-dihydrojodid.

Ein Gemisch aus 0,91 g (2 mmol) 7-[2-(2-Aminothiazol-4-yl)-2-syn-methoxyimino-acetamido]-cephalosporansäure, 2,87 g (16 mmol) Phenanthridin und 2 ml (14 mmol) Trimethyljodsilan in 20 ml Methylendichlorid wird 2 Stunden unter Rückfluß erhitzt. Die Mischung wird gekühlt, ein gelber kristalleiner Niederschlag von Phenanthridinhydrojodid abgesaugt und das Filtrat im Vakuum eingeengt. Hierbei bildet sich ein Niederschlag, der abgesaugt und nacheinander mit Methylendichlorid, Wasser und Äther gewaschen wird. Nach dem Trocknen über $P_2O_5$ erhält man 930 mg (81 % d. TH) der Titelverbindung in Form hellgelber Kristalle.

IR/KBr: 1785 $cm^{-1}$ (Lactam-CO)

$^1H$-NMR($CF_3CO_2D$): $\delta$ = 3,47 und 3,73 (AB, J = 18 Hz, 2H, $SCH_2$); 4,48 (s, 3H, $OCH_3$); 5,25 und 6,40 (AB, J = 7Hz, 2H, $CH_2$-Phenanthridin); 5,40 und 6,03 (AB, J = 5Hz, 2H, 2 Lactam-H); 7,90 - 8,70 (m, 7H, Phenanthridin und Thiazol); 8,80 - 9,30 (m, 2H, 1-H und 10-H von Phenanthridin); 9,88 ppm (s, 1H, 6-H von Phenanthridin).

Analog Beispiel 1, Variante a, werden die nachfolgend aufgeführten Verbindungen in Form der freien Basen erhalten, die der allgemeinen Formel I' entsprechen.

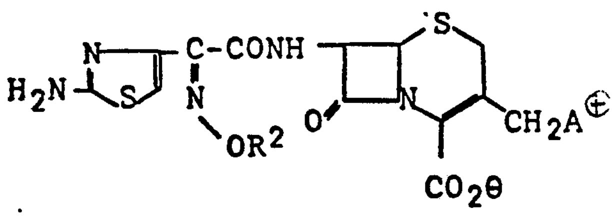

(I')

Die nach Hydrolyse der Reaktionslösung erhaltenen rohen Hydrojodidsalze werden unter Zusatz von Natriumbicarbonat in Lösung gebracht und über Kieselgel (Merck 0.063-0.2 mm) mit Aceton : Wasser (3 : 1) chromatographiert. Nach Gefriertrocknen der Produktfraktionen werden die Verbindungen der Beispiele 6 - 20 in amorpher Form erhalten.

**Tabelle 1:** Verbindungen

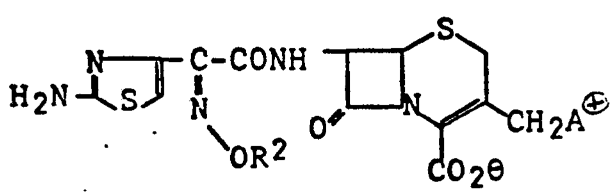

| Beispiel | $R^2$ | A | Ausbeute % d. TH | $^1H$-NMR($CF_3CO_2D$): $\delta$ d(ppm) |
|---|---|---|---|---|
| 6 | $C_2H_5$ | 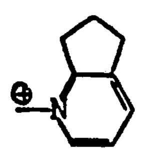 | 20 | 1,43 (t,J = 7Hz, 3H, $CH_2CH_3$); 2,2 - 2,8 (m, 2 Cyclopenten-H); 3,05 - 3,95 (m, 6H, 4 Cydopenten-H |

| | | | | |
|---|---|---|---|---|
| | | | | und $SCH_2$); 4,51 (q, J = 7Hz, 2H, $\underline{CH_2}CH_3$); 5,05 - 6,26 (m, 4H, $CH_2$Py und 2 Lactam-H); 7,42 (s, 1H, Thiazol); 7,5 - 8,7 (m, 3H, Py). |
| 7 | $C_3H_7$ | 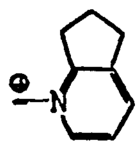 | 46 | 1,08 (t, J = 6Hz, 3H, $CH_3$); 1,6 - 2,8 (m, 4H, $CH_2$ und 2 Cyclopenten-H); 3,1 - 4,2 (m, 6H, 4 Cyclopenten-H und $SCH_2$); 4,53 (t, J = 6Hz, $OCH_2$); 5,1 - 6,3 (m, 4H, $CH_2$Py und 2 Lactam-H); 7,42 (s, 1H, Thiazol); 7,4 - 8,5 ppm (m, 3H, Py). |
| 8 | $CH(CH_3)_2$ | 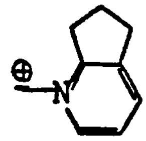 | 37 | 1,47 und 1,57 (d, J = 6Hz, 6H, 2 $CH_3$); 2,1 - 2,8 (m, 2H, Cyclopenten-H); 3,0 - 4,2 (m, 6H, 4 Cyclopenten-H und $SCH_2$); 4,8 (m, 1H, CH); 5,1 - 6,4 (m, 4H, $CH_2$Py und 2-Lactam-H); 7,41 (s, 1H, Thiazol); 7,5 - 8,6 ppm (m, 3H, Py). |
| 9 | $CH_3$ | 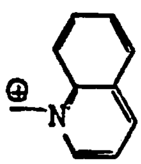 | 36 | 3,40 und 3,80 (AB,J = 19Hz, 2H, $SCH_2$); 4,21 (s, 3H, $OCH_3$); 5,30 - 6,50 (m, 4H, 3-$CH_2$ und 2 Lactam-H mit je 1 d bei 5,41 und 6,10, J = 5Hz, C6 bzw. C7-H); 7,42 (s, 1H, Thiazol); 7,95 - 8,65 (m, 5H, Chinolin-H); 8,95 - 9,40 ppm (m, 2H, Chinolin-H). |
| 10 | $CH_3$ | 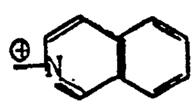 | 36 | 3,45 und 3,93 (AB,J = 18Hz, 2H, $SCH_2$); 4,21 (s 3H, $OCH_3$); 5,25 - 6,50 (m, 4H, 3-$CH_2$ und 2 Lactam-H); 7,41 (s, 1H, Thiazol); 7,95 - 8,80 (m, 6H, Isochinolin-H); 9,79 ppm (bs, 1H, Isochinolin-H). |
| 11 | $CH_3$ | 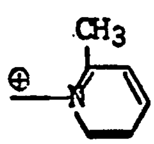 | 33 | 2,98 (s, 3H, $PyCH_3$); 3,52 und 3,71 (AB, J = 19Hz, 2H, $SCH_2$); 4,24 (s, 3H, $OCH_3$); 5,35 - 6,12 (m, 4H, $CH_2$Py und 2 Lactam-H); 7,37 (s, 1H, Thiazol); 7,79 - 8,76 (m, 4H, Py). |
| 12 | $CH_3$ | 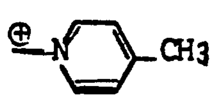 | 42 | 2,80 (s, 3H, $PyCH_3$); 3,45 und 3,85 (AB, J = 19Hz, 2H, $SCH_2$); 4,25 (s, 3H, $OCH_3$); 5,15 - 6,35 (m, 4H, $CH_2$Py und 2 Lactam-H); 7,42 (s, 1H, Thiazol); 7,86 und 8,74 (je ein d, J = 6Hz, 4 Py-H). |

| | | | | |
|---|---|---|---|---|
| 13 | $CH_3$ | 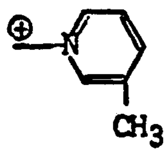 | 67 | 2,60 (s, 3H, $PyCH_3$); 3,63 und 3,86 (AB, J = 19Hz, 2H, $SCH_2$); 4,24 (s, 3H, $OCH_3$); 5,15 - 6,55 (m, 4H, $CH_2Py$ und 2 Lactam-H); 7,42 (s, 1H, Thiazol); 7,75 - 9,05 (m, 4H, Py). |
| 14 | $CH_3$ | 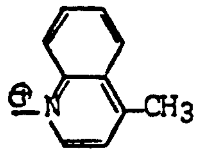 | 19 | 3,15 (s, 3H, $CH_3$); 3,3 und 3,7 (AB, 2H, $SCH_2$); 4,25 (s, 3H, $OCH_3$); 5,1 - 6,7 (m, 4H, $CH_2Py$ und 2 Lactam-H); 7,4 (s, 1H, Thiazol); 7,8 - 9,2 (m, 6H, Lepidin). |
| 15 | $CH_3$ | 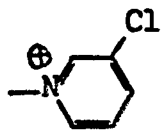 | 26 | 3,50 und 3,85 (AB, J = 19Hz, 2H, $SCH_2$); 4,23 (s, 3H, $OCH_3$); 5,10 - 6,42 (m, 4H, $CH_2Py$ und 2 Lactam-H); 7,40 (s, 1H, Thiazol); 7,90 - 9,15 (m, 4H, Py). |
| 16 | $H_3$ | 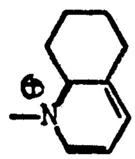 | 68 | 1,7 - 2,4 (m, 4H, Cyclohexen-H); 2,7 - 3,5 (m, 4H, Cyclohexen-H); 3,50 und 3,70 (AB, J = 19Hz, 2H, $SCH_2$); 4,25 (s, 3H, $OCH_3$); 5,38 (d, J = 5Hz, $C_6$-H); 5,55 und 5,80 (AB, 2H, $CH_2Py$); 6,08 (d, J = 5Hz, $C_7$-H); 7,39 (s, 1H, Thiazol); 7,65 - 8,58 (m, 3H, Py). |
| 17 | Ch3 | 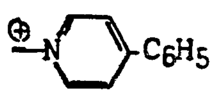 | 55 | 3,55 und 3,83 (AB, J = 19Hz, 2H, $SCH_2$); 4,20 (s, 3H, $OCH_3$); 5,20 - 6,26 (m, 4H, $CH_2Py$ und 2 Lactam-H); 7,35 (s, 1H, Thiazol); 7,51 - 7,89 (m, 5H, $C_6H_5$); 8,26 und 8,91 (AA'BB', J = 7Hz, 4H, Py). |
| 18 | $CH_3$ | 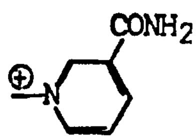 | 50 | 3,60 und 3,83 (AB, J = 18Hz, 2H, $SCH_2$); 4,22 (s, 3H, $OCH_3$); 5,33 - 6,38 (m, 4H, $CH_2Py$ und 2 Lactam-H); 7,36 (s, 1H, Thiazol); 8,12 - 9,60 (m, 4H, Py). |
| 19 | $CH_3$ | 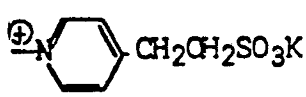 | 17 | 3,4 - 3,9 (m, 6H, $SCH_2$ und $CH_2CH_2$); 4,25 (s, 3H, $OCH_3$); 5,1 - 6,25 (m, 4H, $CH_2Py$ und 2 Lactam-H); 7,38 (s, 1H, Thiazol); 8,12 und 8,79 (AA'BB', J = 6Hz, 4H, Py). |
| 20 | $CH_2COOH$ | 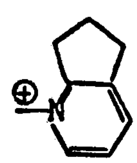 | 15 | 2,3 - 2,8 (m, 2H, Cyclopenten-H); 3,1 - 4,0 (m, 6H, 4 Cyclopenten -H und $SCH_2$); 5,09 (s, 2H, $OCH_2$); 5,1 - 6,25 (m, 4H, $CH_2Py$ und 2 Lactam-H); 7,43 (s, 1H, Thiazol); 7,65 - 8,65 (m, 3H, Py). |

**Beispiel 21**

7-[2-(2-Amino-5-chlorthiazol-4-yl)-2-syn-methoxyimino-acetamido]-3-[2,3-cyclopenteno-1-pyridinio)methyl]-ceph-3-em-4-carboxylat.

Analog Beispiel 1a aus 7-[2-(2-Amino-5-chlorthiazol-4-yl)-2-syn-methoxyiminoacetamido]-cephalosporansäure und 2,3-Cyclopentenopyridin in 66 % Ausbeute erhalten.

$^1H$-NMR ($CF_3CO_2D$): δ = 2,2 - 2,8 (m, 2H, Cyclopenten-H); 3,1 - 4,2 (m, 6H, 4 Cyclopenten-H und $SCH_2$); 4,21 (s, 3H, $OCH_3$); 5,2 - 6,2 (m, 4H, $CH_2Py$ und 2 Lactam-H); 7,6 - 8,6 (m, 3H, Py).

**Beispiel 22**

7-[2-(5-Amino-1,2,4-thiadiazol-3-yl)-2-syn-methoxyimino-acetamido]-3-[(2,3-cyclopenteno-1-pyridinio)methyl]-ceph-3-em-4-carboxylat.

Ein Gemisch aus 46 mg (0,1 mmol) 7-[2-(5-Amino-1,2,4-thiadiazol-3-yl)-2-syn-methoxyimino-acetamido]-cephalosporansäure, 0,1 ml (0,85 mmol) 2,3-Cyclopentenopyridin, 0,1 ml (0,7 mmol) Trimethyljodsilen und 1,5 ml Methylendichlorid wird 1,5 Stunden unter Rückfluß gekocht. Das Lösungsmittel wird im Vakuum entfernt, zum Rückstand 1,5 ml Eiswasser zugegeben und die Lösung über eine Lobar-B-Fertigsäule (Fa. Merck, Darmstadt, Art. 10401) mit Aceton : Wasser (2 : 1) chromatographiert. Die Produktfraktionen werden eingeengt und gefriergetrocknet.

Ausbeute: 32 mg (61,6 %) farbloses amorphes Produkt.

IR (KBr): 1770 $cm^{-1}$ (Lactam-CO)

$^1H$-NMR ($CF_3CO_2D$): δ = 2,25 - 2,85 (m, 2H, Cyclopenten-H); 3,1 - 4,05 (m, 6H, 4 Cyclopenten-H und $SCH_2$); 4,30 (s, 3H, $OCH_3$), 5,2 - 6,2 (m, 4H, $CH_2Py$ und 2 Lactam-H); 7,66 - 8,0 (m, 1Py-H); 8,16 - 8,7 ppm (m, 2H, Py).

Analog Beispiel 1, Variante a, werden die nachfolgend aufgeführten Verbindungen in Form der freien Basen erhalten.

**Tabelle 2:** Verbindungen

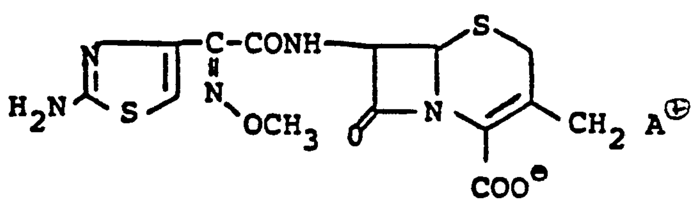

| Beispiel | A | Ausbeute % d. Th. | $^1H$-NMR($CF_3CO_2D$): δ (ppm) |
|---|---|---|---|
| 23 | 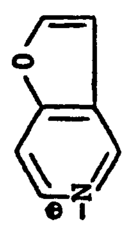 | 52 | 3,45 und 3,85 (AB, J = 19 Hz, 2H, $SCH_2$), 4,20 (s, 3H, $OCH_3$); 5,2 - 6,4 (m, 4H, $CH_2N$ und 2 Lactam-H mit je ein d bei 5,39 und 6,08, J = 5 Hz); 7,30 (m, 1H); 7,40 (s, 1H, Thiazol); 8,0 - 8,3 (m, 2H); 8,7 - 9,0 (m, 1H); 9,43 (bs, 1H/Py). |
| 24 | 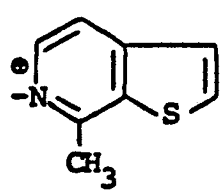 | 43 | 3,28 (s, 3H, $CH_3$); 3,45 und 3,72 (AB, J = 19 Hz, 2H, $SCH_2$); 4,23 (s, 3H, $OCH_3$); 5,25 - 6,4 (m, 4H, $CH_2N$ und 2 Lactam-H mit je ein d bei 5,40 und 6,08, J = 5 Hz); 7,41 (s, 1H, Thiazol); 7,6 - 8,7 (m, 4H/Py). |
| 25 | 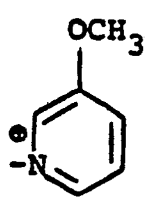 | 82 | 3,48 und 3,81 (AB, J = 19Hz, |

| | | | |
|---|---|---|---|
| d | | | 2H, $SCH_2$); 4,10 und 4,23 (je ein s, 6H, 2 x $OCH_3$) 5,15 - 6.40 (m, 4H, $CH_2N$ un 2 Lactam-H); 7,40 (s, 1H, Thiazol,); 7,9 - 8,8 (m, 4H, Py). |
| 26 | 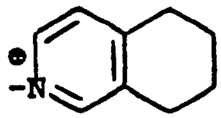 | 42 | 1,8 - 2,3 (m, 4H, Cyclohexen-H); 2,7 - 3,7 (m, 6H, $SCH_2$ und Cyclohexen-H); 4,22 (s, 3H, $OCH_3$); 5,1 - 6,4 (m, 4H, $CH_2N$ und 2 Lactam-H); 7,41 (s, 1H, Thiazol); 7,6 - 8,7 (m, 3H, Py). |

**Patentansprüche**

1. Verfahren zur Herstellung von Cephemverbindungen der allgemeinen Formel I

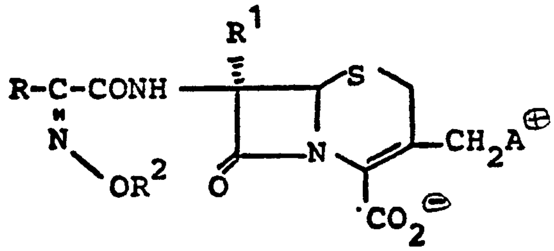

(I)

worin

R einen Thiazolylrest N FIGFIG

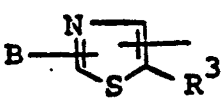

oder einen 1,2,4-Thiadiazolylrest FIGFIG

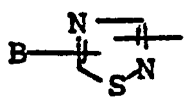

bedeutet, in denen

$R^3$ für Wasserstoff oder Halogen und

B für eine gegebenenfalls substituierte Aminogruppe steht,

und worin

$R^1$ Wasserstoff oder Methoxy,

$R^2$ Wasserstoff, gegebenenfalls substituiertes $C_1$-$C_6$-Alkyl, gegebenenfalls substituiertes $C_2$-$C_6$-Alkenyl, $C_2$-$C_6$-Alkinyl, $C_3$-$C_7$-Cycloalkyl, $C_3$-$C_7$-Cycloalkyl-$C_1$-$C_6$-alkyl, $C_4$-$C_7$-Cycloalkenyl, die Gruppe

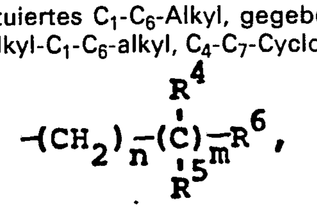

worin m und n jeweils für 0 oder 1 steht,

$R^4$ und $R^5$ gleich oder verschieden sein können und Wasserstoff, Aryl oder eine $C_1$-$C_4$-Alkylgruppe bedeuten, oder zusammen mit dem Kohlenstoffatom, an das sie gebunden sind, eine Methylen- oder eine $C_3$-$C_7$-Cycloalkylidengruppe bilden, wobei die $C_1$-$C_4$-Alkyl- und $C_3$-$C_7$-Cycloalkylidengruppe noch weiter ein- oder mehrfach substituiert sein können,

$R^6$ eine COOH-, CN- oder $CONH_2$-Gruppe, wobei letztere am Stickstoff ein oder zweimal substituiert sein kann,

A einen Chinolinium 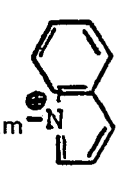 oder einen Isochinoliniumrest

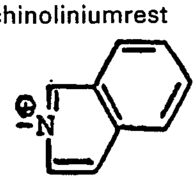 ,

die jeweils auch ein- oder mehrfach, gleich oder verschieden substituiert sein können durch gegebenenfalls substituiertes $C_1$-$C_6$-Alkyl, $C_1$-$C_6$-Alkoxy, Halogen, Trifluormethyl oder Hydroxy; oder einen Phenanthridiniumrest, oder einen Pyridiniumrest

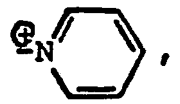

der ein- oder mehrfach, gleich oder verschieden substituiert sein kann durch gegebenenfalls substituiertes $C_1$-$C_6$-Alkyl, wobei 2 orthoständige Alkylgruppen auch zu einem gegebenenfalls substituierten Di- bis Decamethylen-Ring geschlossen sein können, in dem ein Ring-C-Atom durch ein Heteroatom ersetzt sein kann und weiterhin auch noch eine oder zwei Doppelbindungen enthalten sein können; durch gegebenenfalls substituiertes $C_2$-$C_6$-Alkenyl, durch $C_2$-$C_6$-Alkinyl, durch $C_3$-$C_7$-Cycloalkyl oder $C_3$-$C_7$-Cycloalkyl-methyl, wobei in beiden Substituenten der Ring auch substituiert sein kann; durch $C_4$-$C_7$-Cycloalkenyl, durch gegebenenfalls substituiertes $C_1$-$C_6$-Alkoxy, durch $C_2$-$C_6$-Alkenyloxy oder $C_2$-$C_6$-Alkinyloxy, durch Halogen, Trifluormethyl oder Hydroxy, durch gegebenenfalls substituiertes Phenyl, Benzyl oder Heteroaryl,

durch Formyl oder ketalisiertes Formyl,

durch gegebenenfalls substituiertes $C_1$-$C_6$-Alkylcarbonyl, das auch in ketalisierter Form vorliegen kann,

durch Arylcarbonyl, durch Carbamoyl bedeuten und in der die $R^2O$-Gruppe in syn-Position steht, durch Umsetzung eines 3-Jodmethylcephalosporins mit einem Pyridinderivat,

dadurch gekennzeichnet, daß man in einem Eintopf-Verfahren ohne Bildung des 3-Jodmethylderivats eine Verbindung der allgemeinen Formel II

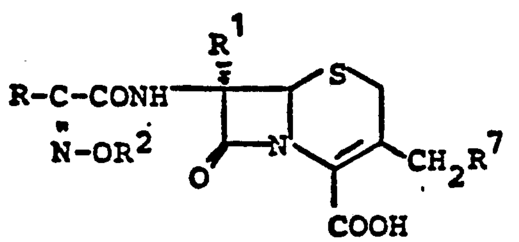

(II)

oder deren Salze, worin

R, $R^1$ und $R^2$ die in der allgemeinen Formel I genannte Bedeutung haben, und

$R^7$ eine Acetoxy-, Chloracetoxy-, Acetylacetoxy- oder Carbamoyloxygruppe bedeutet, mit einer Base, die den Resten A der allgemeinen Formel I entspricht und in Gegenwart von Tri-$C_1$-$C_4$-alkyljodsilan umsetzt und

a) eine gegebenenfalls vorhandene Schutzgruppe abspaltet und

b) falls erforderlich, das so erhaltene Produkt in ein physiologisch verträgliches Säureadditionssalz überführt.

2. Verfahren nach Anspruch 1, dadurch gekennzeichnet, daß das Tri-$C_1$-$C_4$-alkyljodsilan Trimethyl- oder Triethyljodsilan ist.

**Claims**

1. A process for the preparation of a cephem compound of the formula I

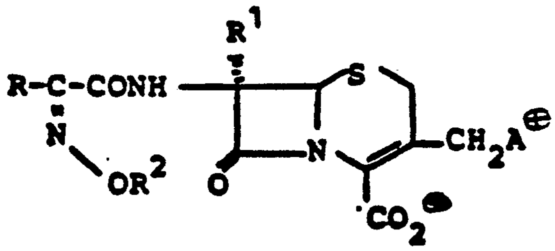

(I)

wherein

R denotes a thiazolyl radical

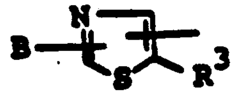

or a 1,2,4-thiadiazolyl radical

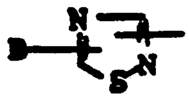

in which
$R^3$ represents hydrogen or halogen and
B represents an optionally substituted amino group, and wherein
$R^1$ denotes hydrogen or methoxy,
$R^2$ denotes hydrogen, optionally substituted $C_1$-$C_6$-alkyl, optionally substituted $C_2$-$C_6$-alkenyl, $C_2$-$C_6$-alkynyl, $C_3$-$C_7$-cycloalkyl,

$C_3$-$C_7$-cycloalkyl-$C_1$-$C_6$-alkyl, $C_4$-$C_7$-cycloalkenyl or the group $-(CH_2)_n-(\overset{R^4}{\underset{R^5}{C}})_m-R^6$,

in which m and n are each 0 or 1, $R^4$ and $R^5$ can be identical or different and denote hydrogen, aryl or a $C_1$-$C_4$-alkyl group, or, together with the carbon atom to which they are bonded, form a methylene or $C_3$-$C_7$-cycloalkylidene group, it being possible for the $C_1$-$C_4$-alkyl and the $C_3$-$C_7$-cycloalkylidene group also to be further monosubstituted or polysubstituted, and
$R^6$ denotes a COOH, CN or $CONH_2$ group, it being possible for the latter to be monosubstituted or disubstituted on the nitrogen, and
A denotes a quinolinium

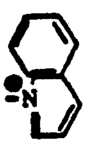

or an isoquinolinium

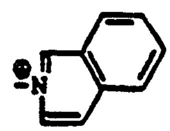

radical, each of which can also be monosubstituted or polysubstituted by identical or different substituents from the group comprising optionally substituted $C_1$-$C_6$-alkyl, $C_1$-$C_6$-alkoxy, halogen, trifluoromethyl and hydroxyl, or denotes a phenanthridinium radical, or denotes a pyridinium radical

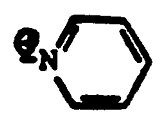,

which can be monosubstituted or polysubstituted by identical or different substituents from the group comprising optionally substituted $C_1$-$C_6$-alkyl, it being possible for 2 alkyl groups in the ortho-position also to be linked to form an optionally substituted di- to deca-methylene ring in which one ring carbon atom can be replaced by a heteroatom and which can furthermore also contain one or two double bonds; by optionally substituted $C_2$-$C_6$-alkenyl, by $C_2$-$C_6$-alkynyl, by $C_3$-$C_7$-cycloalkyl or $C_3$-$C_7$-cycloalkylmethyl, it being possible for the ring in these last two substituents also to be substituted; by $C_4$-$C_7$-cycloalkenyl, by optionally substituted $C_1$-$C_6$-alkoxy, by $C_2$-$C_6$-alkenyloxy or $C_2$-$C_6$-alkynyloxy, by halogen, trifluoromethyl or hydroxyl, by optionally substituted phenyl, benzyl or heteroaryl, by formyl or ketalized formyl, by optionally substituted $C_1$-$C_6$-alkylcarbonyl, which can also be in ketalized form, by arylcarbonyl and by carbamoyl,
and in which the $R^2O$ group is in the syn-position, by reacting a 3-iodomethylcephalosporin with a pyridine derivative, which comprises reacting, in a one-pot process without formation of the 3-iodomethyl derivative, a compound of the formula II

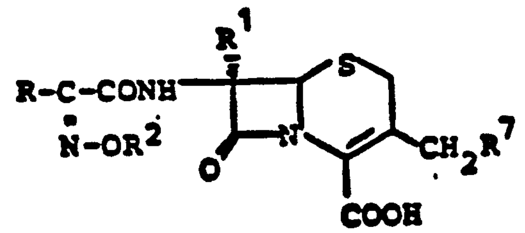

(II)

or salts thereof, in which

R, $R^1$ and $R^2$ have the meaning given formula I and

$R^7$ denotes an acetoxy, chloroacetoxy, acetylacetoxy or carbamoyloxy group,

with a base corresponding to the radicals A of formula I, and in the presence of a tri-$C_1$-$C_4$-alkyliodosilane, and

a) splitting off any protective group present and

b) if necessary, converting the resulting product into a physiologically acceptable acid addition salt.

2. The process as claimed in claim 1, wherein the tri-$C_1$-$C_4$-alkyliodosilane is trimethyl- or triethyl-iodosilane.

**Revendications**

1. Procédé pour la préparation de composés céphème de formule générale I,

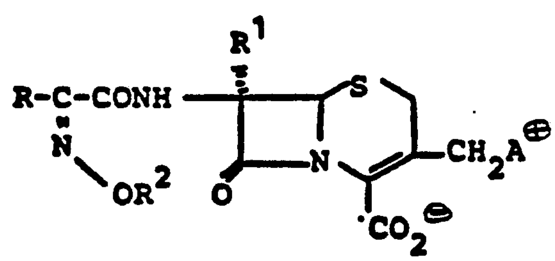

(I)

dans laquelle

R représente un radical thiazolyle 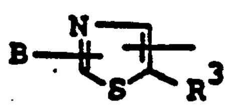

ou un radical 1,2,4-thiadiazolyle 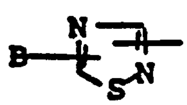 dans lesquels

$R^3$ représente un atome d'hydrogène ou d'halogène, et

B représente un groupe amino éventuellement substitué,

et dans laquelle

$R^1$ représente un atome d'hydrogène ou le groupe méthoxy;

$R^2$ représente un atome d'hydrogène ou un groupe alkyle en $C_1$-$C_6$ éventuellement substitué, alcényle en $C_2$-$C_6$ éventuellement substitué, alcynyle en $C_2$-$C_6$, cycloalkyle en $C_3$-$C_7$, cycloalkyl($C_3$-$C_7$)-alkyle($C_1$-$C_6$), cycloalcényle en $C_4$-$C_7$, le groupe

$$-(CH_2)_n-(\overset{R^4}{\underset{R^5}{C}})_m-R^6,$$

dans lequel

m et n valent chacun 0 ou 1,

$R^4$ et $R^5$ peuvent être identiques ou différents, et représentent un atome d'hydrogène, un groupe aryle ou un groupe alkyle en $C_1$-$C_4$, ou forment, conjointement avec l'atome de carbone auquel ils sont liés, un groupe méthylène ou un groupe cycloalkylidène en $C_3$-$C_7$, le groupe alkyle en $C_1$-$C_4$ et le groupe cycloalkylidène en $C_3$-

$C_7$ pouvant être encore substitués une ou plusieurs fois,

$R^6$ représente un groupe COOH, CN ou $CONH_2$, ce dernier pouvant être une ou deux fois substitué à l'azote;

A représente un radical quinolinium

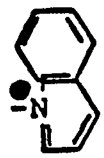

ou un radical isoquinolinium

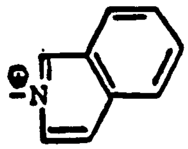

qui peuvent être encore substitués chacun une ou plusieurs fois, de façon identique ou différente, par des halogènes ou par des groupes trifluorométhyle, hydroxy ou alkyle en $C_1$-$C_6$ éventuellement substitué ou alcoxy en $C_{1-6}$ éventuellement substitué; ou un radical phénanthridinium ou un radical pyridinium

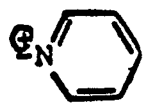

qui peut être une ou plusieurs fois substitué, de façon identique ou différente, par des groupes alkyle en $C_1$-$C_6$ éventuellement substitués, deux groupes alkyle en position ortho pouvant également être cyclisés en un cycle di- à décaméthylène éventuellement substitué, dans lequel un atome de carbone nucléaire peut être remplacé par un hétéroatome, et en outre encore une ou deux doubles liaisons peuvent aussi être présentes; par un groupe alcényle en $C_2$-$C_6$ éventuellement substitué, par un groupe alcynyle en $C_2$-$C_6$, par un groupe cycloalkyle en $C_3$-$C_7$ ou par un groupe cycloalkyl($C_3$-$C_7$)-méthyle, le noyau dans les deux substituants pouvant en outre être substitué; par un groupe cycloalcényle en $C_4$-$C_7$, par un groupe alcoxy en $C_1$-$C_6$ éventuellement substitué, par un groupe alcényloxy en $C_2$-$C_6$ ou alcynyloxy en $C_2$-$C_6$, par des atomes d'halogène, par un groupe trifluorométhyle ou hydroxy, par un groupe phényle, benzyle ou hétéroalkyle éventuellement substitué,

par le groupe formyle ou un groupe formyle sous forme de cétal,

par un groupe alkyl($C_1$-$C_6$)-carbonyle éventuellement substitué, qui peut également être présent sous forme de cétal,

par un groupe arylcarbonyle,

par un groupe carbamyle,

et dans laquelle le groupe $R^2O$ est en position syn, par réaction d'une 3-iodométhylcéphalosporine avec un dérivé de pyridine, caractérisé en ce que l'on fait réagir, dans un procédé en une étape, sans formation du dérivé 3-iodométhyle, un composé de formule générale II

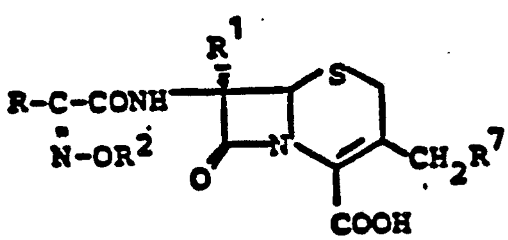

(II)

ou des sels de celui-ci, dans laquelle formule,

R, $R^1$ et $R^2$ ont les significations données dans la formule générale I, et

$R^7$ représente un groupe acétoxy, chloracétoxy, acétylacétoxy ou carbamyloxy,

avec une base qui correspond aux radicaux A de la formule générale I, et en présence d'un trialkyl-($C_1$-$C_4$)-iodosilane, et

a) on élimine un groupe protecteur éventuellement présent, et

b) si nécessaire, on convertit le produit ainsi obtenu en un sel d'addition avec un acide physiologiquement acceptable.

2. Procédé selon la revendication 1, caractérisé en ce que le trialkyl($C_1$-$C_4$)-iodosilane est le triméthyl- ou triéthyliodosilane.